(19) **Europäisches Patentamt / European Patent Office / Office européen des brevets**

(11) **EP 3 554 462 B1**

(12) **FASCICULE DE BREVET EUROPEEN**

(45) Date de publication et mention de la délivrance du brevet:
**25.03.2026 Bulletin 2026/13**

(21) Numéro de dépôt: **17822400.2**

(22) Date de dépôt: **12.12.2017**

(51) Classification Internationale des Brevets (IPC):
**A61K 8/31** (2006.01)   **A61Q 17/04** (2006.01)
**A61Q 5/00** (2006.01)   **A61Q 19/00** (2006.01)
**A61K 8/06** (2006.01)   **A61Q 17/00** (2006.01)
**A61P 17/00** (2006.01)

(52) Classification Coopérative des Brevets (CPC):
**A61K 8/31; A61K 8/062; A61P 17/00; A61Q 5/00; A61Q 17/04; A61Q 19/00;** A61K 2800/59

(86) Numéro de dépôt international:
**PCT/FR2017/053508**

(87) Numéro de publication internationale:
**WO 2018/109354 (21.06.2018 Gazette 2018/25)**

(54) **PROCÉDÉ POUR AMÉLIORER DES PROPRIÉTÉS SENSORIELLES D'ÉMULSIONS HUILE-DANS-EAU**

VERFAHREN ZUR VERBESSERUNG DER SENSORISCHEN EIGENSCHAFTEN VON ÖL-IN-WASSER-EMULSIONEN

METHOD FOR IMPROVING THE SENSORIAL PROPERTIES OF OIL-IN-WATER EMULSIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.12.2016 FR 1662650**

(43) Date de publication de la demande:
**23.10.2019 Bulletin 2019/43**

(73) Titulaires:
• **Société d'Exploitation de Produits pour les Industries Chimiques SEPPIC**
  **75007 Paris (FR)**
• **TotalEnergies OneTech**
  **92400 Courbevoie (FR)**

(72) Inventeurs:
• **CAMBOS, Sophie**
  **81105 Castres (FR)**
• **CLEMENCEAU, Florence**
  **81100 Castres (FR)**
• **MERAT, Emmanuelle**
  **81440 Lautrec (FR)**
• **TAILLEBOIS, Cécile**
  **81990 Salies (FR)**
• **MIZAEL, Sabrina**
  **92800 Puteaux (FR)**
• **STOLTZ, Corinne**
  **94320 Thiais (FR)**
• **MICHEL, Nelly**
  **94700 Maisons Alfort (FR)**
• **SWOBODA, Benjamin**
  **78630 Orgeval (FR)**

(74) Mandataire: **Alatis**
  **3, rue Paul Escudier**
  **75009 Paris (FR)**

(56) Documents cités:
**WO-A2-2008/155060**

• **AGRANA/AAK SWEDEN AB: "Winter Comfort Eco-conscious Hand Cream MAISITA 9040", 24 October 2016 (2016-10-24), XP002770404, Retrieved from the Internet <URL:http://www.agrana.com/de/downloadcenter/?layout=thumbs&id=3739&no_cache=1&itemsPerPage=36> [retrieved on 20170516]**

**(Cont. page suivante)**

- **JENNIFER NOVOSELETSKY: "Seppic introduces Emollient Ranges by the Double", 17 April 2016 (2016-04-17), XP002770405, Retrieved from the Internet <URL:http://www. cosmeticsandtoiletries.com/formulating/ category/skincare/ Seppic-Introduces-Emollient-Ranges-by-the-Double-375459721.html> [retrieved on 20170516]**

**Description**

L'invention a pour objet un nouveau procédé d'amélioration des propriétés sensorielles et esthétiques d'une émulsion de type huile-dans-eau sur la peau humaine.

Un nombre important de formulations cosmétiques à application topique ont pour but de protéger la peau, les muqueuses et le cuir chevelu, des agressions et des stresses extérieurs et environnementaux. Par exemple, le consommateur recherche des formulations à appliquer sur la peau qui le protégera des effets néfastes et inesthétiques d'une exposition prolongée aux rayonnements ultra-violets du soleil, ou qui préviendra contre des altérations à l'intégrité de sa peau suite à une exposition de plus en plus fréquentes aux agents polluants présents dans les atmosphères, et plus particulièrement les atmosphères urbaines.

Pour répondre à ces demandes visant à maintenir et/ou à restaurer l'intégrité de la peau face à des éléments extérieurs identifiés ou au vieillissement naturel, les industries de la cosmétique et de la dermocosmétique ont développés ces dernières décennies de nouveaux ingrédients spécifiques pour améliorer les performances requises par les consommateurs.

D'autre part, le consommateur est aussi en attente de propriétés d'ordres sensorielles et esthétiques apportées par les compositions cosmétiques et dermocosmétiques, qui lui procure à la fois des sensations de bien-être pendant et après l'application sur la peau, et également une identification à un aspect extérieur de la formulation cosmétique ou dermocosmétique, renvoyant à un critère de qualité. Ainsi, les consommateurs sont en recherche de compositions cosmétiques ou dermocosmétique dont la forte consistance que l'on nomme « richesse » est souvent associée à un sentiment de confort cutané pendant et après l'application sur la peau. Cette sensation de confort est renforcée lorsque l'étape d'étalement de la composition cosmétique ou dermo-cosmétique est aisée, à savoir lorsque sa durée est réduite ou plus précisément n'est pas prolongée suite à des résistances sur la peau lors dudit étalement et/ou lorsque le consommateur doit appliquer une force de cisaillement sur la peau élevée, se traduisant également par une vitesse plus élevée d'application à l'étalement et/ou par l'apposition d'une force à intensité plus élevée lors de la dite phase d'étalement.

Dans des cas spécifiques, comme par exemple ceux des formulations cosmétiques ou dermocosmétiques destinées à la prévention contre les effets non désirés (rougeurs, érythèmes et brûlures) des rayonnements ultra-violets du soleil sur la peau ou ceux des crèmes destinées aux traitements anti-inflammatoires et antirhumatismales par voie locale, comprenant par exemple de l'ibuprofène, du camphre, du diclofenac ou des essences de girofle, qui se caractérisent par des difficultés de mise en oeuvre lors de l'étalement, il a été observé que les consommateurs sont moins rigoureux dans la mise en œuvre des procédures d'application desdits produits de protection ou de soin. Ils appliquent ainsi souvent trop peu de produit et/ou en une fréquence pas assez soutenue et il en résulte alors une moindre protection de la peau que celle prévue dans la notice et/ou sur le packaging.

Ainsi, pour encourager une meilleure et plus fréquente application de ces formulations de prévention et de protection de la peau, il est important que les dites formulations présente des propriétés sensorielles agréables et puissent s'étaler sur la peau de façon homogène, rapide et sans impliquer une intensité d'application trop importante.

D'autre part, les consommateurs sont à la recherche de formulations cosmétiques ou dermocosmétiques qui n'altèrent pas l'aspect extérieur de la peau, comme par exemple en laissant un résiduel huileux sous la forme d'un léger film et, *a contrario,* une formulation cosmétique ou dermocosmétique conférant un aspect mat à la peau sera préférée et recherchée.

Enfin, l'aspect extérieur de la formulation cosmétique ou dermocosmétique, peut constituer un critère de choix et d'attraction avéré pour son utilisation par le consommateur. Ainsi, une formulation cosmétique ou dermocosmétique présentant un aspect extérieur hétérogène, laissant apparaitre une légère couche d'huile jaunâtre à la surface après stockage ne sera considérée comme attractive alors qu'au contraire une formulation cosmétique ou dermocosmétique homogène après stockage prolongé et possédant un aspect brillant sera considérée comme attractive car reflétant une image extérieure de qualité.

Prenant en considération toutes ces exigences, la personne du métier ne peut que constater qu'un nombre important de produits de protection et de soin, comme par exemple les produits de protection solaire ou de traitement topique des inflammations, qui montrent une grande efficacité, provoquent cependant des sensations grasses et désagréables lors de leur étalement sur la peau, ledit étalement devant se réaliser dans une durée relativement importante et nécessiter une vitesse d'application plus intense.

Ces formulations de protection et de soins peuvent se présenter sous la forme d'une émulsion de type huile-dans-eau pour pouvoir procurer une sensation plus agréable par la mise au contact de la peau une phase aqueuse, mais les agents émulsionnants tensioactifs ou polymériques nécessaires au maintien de la stabilité de ladite émulsion huile-dans-eau peuvent également contribuer à un inconfort lors de l'application en conférant une sensation de lourdeur à l'émulsion pendant et après son application sur la peau.

Une alternative peut consister en la mise au point d'une émulsion de type eau-dans-huile, laquelle est stabilisée par épaississement de la phase huileuse par la mise en œuvre notamment de cires et d'alcools gras linéaires dont la chaîne hydrocarbonée comprend de 14 à 22 atomes de carbone. On utilise également des élastomères siliconés pour stabiliser une telle émulsion de type eau-dans-huile, mais lesdits élastomères siliconés impliquent également une détérioration des propriétés sensorielles de la peau ainsi traitée par l'application de ladite émulsion. Par ailleurs, l'utilisation de ces élastomères siliconés comme agents stabilisants d'émulsions solaires eau-dans-huile modifie dans certains cas la solubilité des écrans solaires utilisés qui peuvent alors précipiter dans le flacon lors du stockage.

Pour éviter de se trouver confronté aux inconvénients évoqués précédemment à la fois pour les émulsions de type eau-dans-huile et pour les émulsions de type huile-dans-eau, le formulateur doit mettre au point une émulsion présentant des propriétés d'étalement améliorées, permettant alors au consommateur d'appliquer l'émulsion sur sa peau, sans qu'il se trouve confronté à des forces de frictions contraires au mouvement instauré par l'applicateur et ralentisse alors la dite application et/ou nécessite une vitesse d'étalement plus importante.

La demande de brevet européen publiée sous le numéro EP 2 644 188 A1 des émulsions de type huile-dans-eau présentant des propriétés d'étalement sur la peau améliorées comprenant une combinaison d'au moins une résine de silicone réticulée et non émulsionnante, au moins un alcool polyvinylique, un épaississant de type polyacrylamide, une huile choisie parmi les éléments du groupe constitué par les huiles végétales de type triglycéridiques, les cires, les alcools gras éthoxylés, les esters d'acides gras, les acides gras, les alcools gras, les huiles de silicone et les huiles perfluorées.

La demande internationale publiée sous le numéro WO 2011/065771 divulgue et enseigne que l'étalement et la douceur procurée lors de l'application d'une émulsion de type eau-dans-huile-dans-eau, préparée à partir d'une émulsion de type eau-dans-huile comprenant un émulsionnant sur base silicone, émulsionnant de type di-polyhydroxyalkylate, un épaississant minéral de type hectorite, et une huile polaire.

Ces solutions de l'état de la technique décrivent une amélioration des propriétés d'étalement d'une émulsion en mettant en œuvre des composés siliconés et d'autres tensioactifs pour lesquels l'homme du métier recherche une alternative dans une démarche de prise en compte d'un développement durable, et plus particulièrement dans une démarche d'utiliser des ingrédients n'émettant pas et/ou dont la fabrication n'implique pas de composés volatiles organiques (VOC) et/ou d'utiliser des ingrédients biodégradables selon les normes réglementaires en vigueur et/ou d'utiliser des ingrédients d'origine végétale et non plus fossile. Certaines des propriétés esthétiques recherchées, comme par exemple celles visant à conférer un aspect mat sur la peau, sont atteintes par la mise en œuvre dans la formulation cosmétique de polymères considérés comme des matières plastiques et donc pour lesquels une alternative à l'obtention d'un effet mat sur la peau est recherchée.

De façon plus précise, les dérivés de silicone, comme les substances et compositions chimiques de la famille des polysiloxanes est connue pour apporter des propriétés sensorielles améliorées aux émulsions huile-dans-eau et notamment en terme de facilité d'étalement et de limitation de résidus lipides sur la peau après application. Cependant, les caractéristiques environnementales associées à ces ingrédients ont nécessité la recherche de substituts qui procurent des propriétés sensorielles similaires tout en ayant des caractéristiques environnementales conformes aux réglementations en vigueur et à venir, et conformes aux exigences des consommateurs en la matière. Une alternative partiellement satisfaisante a été mise en évidence, par l'utilisation de compositions d'alcanes comprenant des quantités importantes de cyclo-alcanes pour préparer des émulsions cosmétiques huile-dans-eau ; lesdits mélanges d'alcanes présentant des propriétés satisfaisantes de biodégradabilité et des propriétés sensorielles satisfaisantes mais jugées perfectibles.

Par conséquent, il existe un besoin de mettre au point des formulations cosmétiques ou dermocosmétiques qui présentent des propriétés sensorielles améliorées, notamment qui procurent une sensation de richesse et qui soit facile à étaler sur la peau, et qui présentent des caractéristiques esthétiques améliorées et/ou attractives comme par exemple se caractérisant par un aspect brillant amélioré et conférant à la peau un aspect mat et non brillant.

Le document Agrana/AAK Sweden AB : « Winter Comfort Eco-conscious Hand Cream MAISITA 9040" décrit une crème pour les mains dans laquelle grasse comprend un mélange d'hydrocarbures en C15-C19 et du beurre de karité.

Le document Jennifer Novoseletsky « Seppic introduces Emollient Ranges by the Double », www.cosmeticsandtoiletries.com, 17 avril 2016, divulgue que les mélanges d'hydrocarbures Emogreen L15 et Emosmart L19 procurent aux compositions cosmétiques qui les contiennent une large palette sensorielle.

Le document WO 2008/155060 décrit des mélanges d'au moins deux hydrocarbures, ayant des chaines carbonées de longueurs qui diffèrent d'au moins un atome de carbone de préférence comportant de 11 à 21 atomes de carbone et leur utilisation dans des compositions cosmétiques pour leur procurer des propriétés sensorielles et/ou esthétiques améliorées.

Les inventeurs ont donc cherché à développer une nouvelle solution pour améliorer les propriétés sensorielles et esthétiques d'une émulsion de type huile-dans-eau à usage topique, n'employant pas nécessairement de dérivés siliconés, mais mettant en œuvre des compositions chimiques d'origines végétales et/ou biodégradables.

C'est pourquoi selon un premier aspect, l'invention a pour objet un procédé pour améliorer les propriétés sensorielles et/ou esthétiques d'une émulsion $(E_0)$ de type huile-dans-eau exempte de beurre de karité, tel que défini à la revendication 1.

Par « propriétés sensorielles » d'une $(E_0)$ huile-dans-eau, on désigne au sens de la présente invention les sensations éprouvées et constatées par un utilisateur qui s'applique sur la peau l'émulsion (E) de type huile-dans-eau résultant dudit procédé ci-dessus et qui correspondent à des caractéristiques physico-chimiques et/ou rhéologiques de ladite émulsion (E).

Par « propriétés esthétiques» on désigne au sens de la présente invention des caractéristiques visuelles associées à l'émulsion à usage (E) huile-dans-eau en tant que telle et également associées à l'état de la peau après application de ladite émulsion à usage topique (E) huile-dans-eau.

Par quantité efficace, on désigne, dans la définition du procédé tel que défini ci-dessus, une quantité telle, que l'émulsion à usage topique (E) de type huile-dans-eau résultant dudit procédé ci-dessus: montre une valeur moyenne d'au moins trois mesures de la variation du coefficient de friction, en fonction de la vélocité d'application comprise entre 1 radian.s$^{-1}$ et 4 radian.s$^{-1}$, inférieure ou égale à 10 % de la valeur initialement mesurée pour une vélocité d'application sur la peau comprise égale à 1 radian.s$^{-1}$ ; lesdits coefficients de friction étant enregistrés lors de chacune des mesures par l'intermédiaire d'un rhéomètre DHR2 (TA Instrument) équipé d'un support de type plan Peltier sur lequel est posé une surface de plexiglas sur laquelle est déposée l'émulsion à tester.

Par émulsion ($E_0$) de type huile-dans-eau, on désigne au sens de la présente invention les émulsions comprenant pour 100% de leur masse :

- de 95% à 50%, plus particulièrement de 90% à 70% d'une phase aqueuse ($A_0$) cosmétiquement acceptable,
- de 5% à 50%, plus particulièrement de 10% à 30% d'une phase grasse ($G_0$), ladite phase grasse ($G_0$) comprenant pour 100% de sa masse, de 1% à 12%, plus particulièrement de 2% à 8% d'au moins un agent tensioactif de type huile-dans-eau et de 88% à 99%, plus particulièrement de 92% à 98% d'au moins une huile et/ou une cire.

Par « huile » présente dans la phase grasse ($G_0$) de l'émulsion ($E_0$) de type huile-dans-eau telle que définie précédemment, on désigne au sens de la présente invention les substances chimiques ou les mélanges de substances chimiques insolubles dans l'eau, et se présentant sous un aspect liquide à une température de 25°C.

Par « cire » présente dans la phase grasse ($G_0$) de l'émulsion ($E_0$) de type huile-dans-eau telle que définie précédemment, on désigne au sens de la présente invention les substances chimiques ou les mélanges de substances chimiques insolubles dans l'eau, et se présentant sous un aspect solide à une température de 45°C.

Par «agent tensioactif de type huile-dans-eau » présent dans la phase grasse ($G_0$) de l'émulsion ($E_0$) topique de type huile-dans-eau telle que définie précédemment, on désigne au sens de la présente invention, la substance chimique ou le mélange de substances chimiques qui permet de stabiliser les gouttelettes de ladite phase grasse ($G_0$) en dispersion dans la phase continue ($A_0$).

Comme agent tensioactif de type huile-dans-eau présent dans la phase grasse (G°) de l'émulsion ($E_0$) de type huile-dans-eau telle que définie précédemment, on peut citer par exemple :

- Des polysorbates résultant de la réaction d'éthoxylation entre un équivalent molaire d'esters de sorbitan et entre 5 et 20 équivalents molaires d'oxyde d'éthylène, et plus particulièrement entre un équivalent molaire de laurate de sorbitan, ou de palmitate de sorbitan, ou de stéarate de sorbitan, ou d'isostéarate de sorbitan, ou d'oléate de sorbitan, et entre 5 et 20 équivalents molaires d'oxyde d'éthylène ;
- Les produits résultant de la réaction d'éthoxylation entre un équivalent molaire d'un acide gras, comme par exemple l'acide palmitique, l'acide myristique, l'acide laurique, l'acide stéarique, l'acide isostéarique, l'acide oléique, et entre 5 à 40 équivalents molaires d'oxyde d'éthylène ;
- Les produits résultant de la réaction d'estérification entre un acide gras, comme par exemple l'acide palmitique, l'acide myristique, l'acide laurique, l'acide stéarique, l'acide isostéarique, l'acide oléique, l'acide arachidique, l'acide béhénique et entre 4 à 20 équivalents molaires, plus particulièrement entre 3 à 10 équivalents molaires de glycérol.

L'expression "cosmétiquement acceptable" utilisée dans la définition de la phase aqueuse ($A_0$) de l'émulsion de type huile-dans-eau, signifie, selon la directive du Conseil de la Communauté Economique Européenne N°76/768/CEE du 27 juillet 1976 modifiée par la directive N°93/35/CEE du 14 juin 1993, toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état. Un milieu cosmétiquement acceptable de ces compositions objet de l'invention peut contenir classiquement de l'eau, un ou plusieurs solvants organiques cosmétiquement acceptables, un mélange d'eau et d'un ou plusieurs solvants organiques. Les solvants cosmétiquement acceptables peuvent plus particulièrement être choisis parmi les alcools polyhydriques comme par exemple le glycérol, le diglycérol, les oligomères du glycérol, l'éthylène glycol, le propylène glycol, l'hexylène glycol, le diéthylène glycol, le xylitol, l'érythritol, le sorbitol, ou les alcools hydrosolubles.

Selon un mode particulier du procédé tel que défini ci-dessus, par quantité efficace dudit mélange ($M_1$), on désigne une proportion massique de 1% à 25% de l'émulsion huile-dans-eau, tout particulièrement de 5% à 20%.

L'expression "à usage topique" utilisée dans la définition du procédé tel que défini ci-dessus, signifie que ladite composition est mise en œuvre par application sur la peau, les cheveux, le cuir chevelu ou les muqueuses, qu'il s'agisse d'une application directe dans le cas d'une composition cosmétique, dermocosmétique, dermopharmaceutique ou pharmaceutique ou d'une application indirecte par exemple dans le cas d'un produit d'hygiène corporelle sous forme de lingette en textile ou en papier ou de produits sanitaires destinés à être en contact avec la peau ou les muqueuses.

Par « alcanes linéaires » présents dans le mélange ($M_1$) mis en œuvre dans le procédé objet de la présente invention, et comportant de quinze à dix-neuf atomes de carbone, on désigne plus particulièrement au sens de la présente invention les éléments choisis parmi le groupe constitué par le pentadécane, l'hexadécane, l'heptadécane, l'octadécane et le nonadécane.

Par « alcanes ramifiés » présents dans le mélange ($M_1$) mis en œuvre dans le procédé objet de la présente invention, et comportant de quinze à dix-neuf atomes de carbone, on désigne plus particulièrement au sens de la présente invention les éléments choisis parmi le groupe constitué par le 2-méthyl tétradécane (ou isopentadécane), le 2-méthyl pentadécane (ou isohexadécane), le 2-méthyl hexadécane (ou isoheptadécane), le 2-méthyl heptadécane (ou isooctadécane) et le 2-méthyl octadécane (ou isononadécane).

Par cyclo-alcanes présents dans le mélange ($M_1$) mis en œuvre dans le procédé objet de la présente invention, et comportant de 15 à 19 atomes de carbone, on désigne plus particulièrement au sens de la présente invention des hydrocarbures saturés comprenant au moins un groupe hydrocarboné cyclique saturé optionnellement substitué par un ou plusieurs radicaux alkyles linéaires ou ramifies.

Selon un aspect particulier, l'invention a pour objet un procédé tel que défini à la revendication 2.

Selon un aspect plus particulier, l'invention a pour objet un procédé tel que défini précédemment, dans lequel le mélange ($M_1$) est un mélange d'hydrocarbures saturés commercialisé sous le nom de marque Emogreen™L15 comprenant pour 100% de sa masse :

i) 3,7% d'alcanes linéaires comportant de quinze à dix-neuf atomes de carbone,
ii) 96% d'iso-alcanes comportant de quinze à dix-neuf atomes de carbone, et
iii) 0,3% de cyclo-alcanes comportant de quinze à dix-neuf atomes de carbone.

Selon un autre aspect particulier, l'invention a pour objet un procédé tel que défini à la revendication 3.

Selon un aspect tout particulier, l'invention a pour objet un procédé tel que défini précédemment, dans lequel le mélange ($M_1$) est un mélange d'hydrocarbures saturés commercialisé sous le nom de marque Emosmart™L19, comprenant pour 100% de sa masse :

i) 13,20% massique d'alcanes linéaires comportant de 15 à 19 atomes de carbone,
ii) 55,00% massique d'iso-alcanes comportant de 15 à 19 atomes de carbone, et
iii) 31,80% de cyclo-alcanes comportant de 15 à 19 atomes de carbone.

Par «propriétés d'étalement sur la peau », on désigne au sens de la présente invention, la capacité pour l'émulsion huile-dans-eau objet de la présente invention d'être étalée sur la surface de la peau en une mince couche et de façon à couvrir une large surface de la peau tout en restant suffisamment concentré pour remplir la mission qui lui est assignée de par sa composition, comme par exemple la protection de la peau envers les conséquences d'une exposition prolongée aux rayonnements ultra-violets du soleil si l'émulsion huile-dans-eau contient des filtres solaires organiques et/ou inorganique. Les propriétés d'étalement sur la peau d'une émulsion de type huile-dans-eau, à savoir la facilité ou la difficulté d'étalement sur la peau humaine de ladite émulsion, peut être évaluée par la mise en œuvre de différentes méthodes, comme par exemple les méthodes permettant la mesure des valeurs du seuil d'écoulement, et/ou la mesure de l'indice de rhéo-fluidification, et/ou la mesure du coefficient de friction et/ou la mesure de la variation du coefficient de friction en fonction de la rapidité dudit étalement.

Par "richesse" de ladite émulsion à usage topique ($E_0$), on désigne au sens de la présente invention la sensation procurée par l'application sur la peau d'une émulsion à usage topique ($E_0$), telle que définie précédemment, qui se caractérise par une consistance compacte non fluide ou non coulante si soumise à la seule force de la gravité, et une présence de l'émulsion lors de l'étalement sur la peau significativement plus importante en comparaison avec l'étalement de l'eau ; par « présence », la personne du métier entend donc une perception sensorielle dénuée d'un caractère aqueux.

Selon un autre aspect particulier, l'invention a pour objet le procédé tel défini précédemment pour lequel la propriété esthétique est la brillance de l'émulsion à usage topique ($E_0$) de type huile-dans-eau résultant dudit procédé ci-dessus. Par "brillance", on désigne au sens de la présente invention, la capacité de l'émulsion à réfléchir des rayons incidents provenant d'une source de lumière du spectre du visible avec un rendement de réflexion donné.

Selon un autre aspect particulier, l'invention a pour objet le procédé tel défini précédemment pour lequel la propriété esthétique est l'aspect mat de la peau constaté après l'application sur la peau d'une émulsion à usage topique ($E_0$) de type huile-dans-eau résultant dudit procédé ci-dessus. Par "aspect mat de la peau", on désigne au sens de la présente invention, la capacité de la peau à absorber une proportion donnée de rayons incidents provenant d'une source de lumière du spectre du visible, ladite capacité étant liée à l'épaisseur et la nature du film résiduel d'émulsion restant sur la peau après son application.

L'invention a aussi pour objet une émulsion à usage topique de type huile-dans-eau (E) telle que définie à la revendication 6.

Par "phase grasse (G)", on désigne au sens de la présente invention, un corps gras ou un mélange de corps gras insoluble dans l'eau et/ou dans les mélanges d'eau et de solvants polaires. Une telle « phase grasse » peut comprendre des huiles et/ou des cires. Parmi les éléments constitutifs de la phase grasse, on peut citer :

- Les huiles d'origine animale, telles que le squalène ou le squalane;
- les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de mais, l'huile de soja, l'huile de coton, l'huile de luzeme, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, l'huile de noyau d'abricot, l'huile de calophyllum, l'huile de sysymbrium, l'huile d'avocat, l'huile de calendula, l'huile de sésame, l'huile de reine des prés, l'huile de Macadamia kiwi, l'huile de bourrache, l'huile de pépins de cassis, l'huile de café, l'huile de pistache, l'huile de noyau de pêche, l'huile de pépin de framboise, l'huile de pépin de fraise, l'huile de melon, l'huile de pépin de myrtille, l'huile d'argan, l'extrait huileux de prune, l'huile de grenade, l'huile de papaye, l'huile de lait de coco, les huiles issues de fleurs ou de légumes;
- Les huiles végétales éthoxylées;
- Les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les polyalphaoléfines, les polyoléfines comme le polyisobutène, le polydécène hydrogéné ou le polyisobutène hydrogéné, commercialisé en France par la société Ets B. Rossow et Cie sous le nom PARLEAM - POLYSYNLANE™, cité dans: Michel and Irene Ash; Thesaurus of Chemical Products, Chemical Publishing Co, Inc. 1986 Volume I, page 211 (ISBN 0 7131 3603 0).
- Les huiles de silicone comme les diméthylpolysiloxanes, les méthylphényl-polysiloxanes, les silicones modifiées par des amines, les silicones modifiées par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.
- Des cires comme la cire d'abeille, la cire de carnauba, la cire de candelilla, la cire d'ouricoury, la cire du Japon, la cire de fibre de liège, la cire de canne à sucre, la cire de jojoba, la cire de fleurs de cassis, la cire de fleurs de narcisse, la cire de fleurs d'oranger, la cire d'orange, la cire de riz, les cires de paraffines, les cires de lignite, les cires microcristallines, la cire de lanoline; l'ozokérite; la cire de polyéthylène, les cires de silicone; les cires végétales; les alcools gras et les acides gras solides à température ambiante; les glycérides solides à température ambiante.

Par «agent tensioactif de type huile-dans-eau » présent dans la phase grasse (G) de l'émulsion (E) de type huile-dans-eau telle que définie précédemment, une substance chimique ou le mélange de substance chimique qui permet de stabiliser les gouttelettes de la phase grasse (G) en dispersion dans la phase continue (A). On peut citer par exemple :

- Des polysorbates résultant de la réaction d'éthoxylation entre un équivalent molaire d'esters de sorbitan et entre 5 et 20 équivalents molaires d'oxyde d'éthylène, et plus particulièrement entre un équivalent molaire de laurate de sorbitan, ou de palmitate de sorbitan, ou de stéarate de sorbitan, ou d'isostéarate de sorbitan, ou d'oléate de sorbitan, et entre 5 et 20 équivalents molaires d'oxyde d'éthylène;
- Les produits résultant de la réaction d'éthoxylation entre un équivalent molaire d'un acide gras, comme par exemple l'acide palmitique, l'acide myristique, l'acide laurique, l'acide stéarique, l'acide isostéarique, l'acide oléique, et entre 5 à 40 équivalents molaires d'oxyde d'éthylène;
- Les produits résultant de la réaction d'estérification entre un acide gras, comme par exemple l'acide palmitique, l'acide myristique, l'acide laurique, l'acide stéarique, l'acide isostéarique, l'acide oléique, l'acide arachidique, l'acide béhénique et entre 4 à 20 équivalents molaires, plus particulièrement entre 3 à 10 équivalents molaires de glycérol.

Une phase aqueuse cosmétiquement acceptable (A) comprise dans l'émulsion huile-dans-eau (E) telle que définie précédemment peut contenir classiquement un ou plusieurs solvants organiques cosmétiquement acceptables, un mélange d'eau et d'un ou plusieurs solvants organiques cosmétiquement acceptables. Les solvants cosmétiquement acceptables peuvent plus particulièrement être choisis parmi les alcools polyhydriques comme par exemple le glycérol, le diglycérol, le triglycérol, les oligomères du glycérol, le xylitol, l'érythritol, le sorbitol, le méthyl-2-propanediol-1,3; les alcools polyhydriques alcoxylés; les glycols, comme par exemple le butylène glycol, l'hexylène glycol, le caprylyl glycol ou 1,2-octanediol, le pentylène glycol ou 1,2-pentanediol, le monopropylène glycol, le dipropylène glycol, l'isoprène glycol, le butyldiglycol, les polyéthylènes glycols dont le poids moléculaire est compris entre 200g.mol-1 et 8.000g.mol-1; ou les alcools hydrosolubles comme par exemple l'éthanol, l'isopropanol ou le butanol.

Par agent de protection contre les rayonnements ultra-violets du soleil, on désigne notamment dans la définition de l'émulsion de type huile-dans-eau (E) objet de la présente demande brevet, les pigments, les filtres organiques solaires et les filtres inorganiques solaires.

Comme pigments utilisés comme agent de protection contre les rayonnements ultra-violets du soleil, il y a par exemple le dioxyde de titane, les oxydes de fer marrons, les oxydes de fer jaune, les oxydes de fer noir, ou les oxydes de fer rouges ou encore les pigments nacrés blancs ou colorés tels que les Mica-Titane.

Comme filtres organiques solaires utilisés comme agent de protection contre les rayonnements ultra-violets du soleil, il y a par exemple :

- Ceux de la famille des dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA;
- Ceux de la famille des dérivés de l'acide anthranilique comme l'homomenthyl-N-acétyl anthranilate;
- Ceux de la famille des dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle;
- Ceux de la famille des dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl-□α-cyano-β-phényle, le cinnamate de diparaméthoxy mono-2-éthylhexanoyl de glycéryle;
- Ceux de la famille des dérivés de la benzophénone comme la 2,4-dihydroxy benzophénone, la 2,2'-dihydroxy 4-méthoxy benzophénone, la 2,2',4,4'-tétrahydroxy benzophénone, la 2-hydroxy 4-méthoxy benzophénone, la 2-hydroxy 4-méthoxy 4'-méthyl benzophénone, la 2-hydroxy 4-méthoxy benzophénone-5-sulfonate, la 4-phényl benzophénone, le 2-éthylhexyl 4'-phényl benzophénone-2-carboxylate, la 2-hydroxy 4-n-octyloxy benzophénone, la 4-hydroxy 3-carboxy benzophénone ; le 3-(4'-méthylbenzylidène) d,l-camphre, le 3-(benzylidène)-d,l-camphre, le benzalkonium méthosulfate camphre ; l'acide urocanique, l'urocanate d'éthyle ;
- Ceux de la famille des dérivés de l'acide sulfonique comme l'acide 2-phényl benzimidazole-5-sulfonique et ses sels ; la famille des dérivés de la triazine comme l'hydroxyphényl triazine, l'éthylhexyloxyhydroxyphényl-4-méthoxyphényltriazine, le 2,4,6-trianillino-(p-carbo-2'-éthylhexyl-1'-oxy) 1,3,5-triazine, le 4,4-((6-(((1,1-diméthyléthyl) amino) carbonyl) phenyl) amino)-1,3,5-triazine-2,4-diyl diimino) bis-(2-éthylhexyl) ester de l'acide benzoïque, le 2-phényl-5-méthyl benzoxazole, le 2,2'-hydroxy-5-méthylphényl benzotriazole, le 2-(2'-hydroxy-5'-t-octylphényl) benzotriazole, le 2-(2'-hydroxy-5'-méthyphényl) benzotriazole; la dibenzazine; le dianisoylméthane, le 4-méthoxy-4"-t-butylbenzoylméthane ; la 5-(3,3-diméthyl-2-norbornylidène)-3-pentan-2-one ; le 2-(4-diethylamino 2-hydroxy benzoyl) benzoic acid hexyl ester, le 2,4-bis{[4-(2-ethylhexyloxy)-2-hydroxy] phenyl}-6-(4-methoxy phenyl) 1,3,5-triazine, le 2,4,6-tris[4-(2-ethylhexyloxycarbonyl) anilino]-1,3,5-triazine, le 2-ethylhexyl dimethoxybenzylidene dioxoimidazolidine propionate, la famille des dérivés du diphénylacrylate comme le 2-éthylhexyl-2-cyano-3,3-diphényl-2-propènoate, l'éthyl-2-cyano-3,3-diphényl-2-propènoate;
- Ceux de la famille des polysiloxanes comme le malonate de benzylidène siloxane.

Comme filtres inorganiques solaires utilisés comme agent de protection contre les rayonnements ultra-violets du soleil, il y a par exemple : les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

Selon un aspect particulier, l'invention a pour objet une émulsion huile-dans-eau à usage topique (E) telle que définie précédemment, dans laquelle l'agent de protection contre les rayonnements ultra-violets du soleil est choisi parmi les éléments du groupe constitué par le dioxyde de titane, la 2,4-dihydroxy benzophénone, le 2-(4-diethylamino-2-hydroxybenzoyl) benzoic acid hexyl ester, le 2,4-bis{[4-(2-ethylhexyloxy)-2-hydroxy] phenyl}-6-(4-methoxyphenyl)-1,3,5-triazine, le 2,4,6-tris[4-(2-ethylhexyloxy carbonyl) anilino]-1,3,5-triazine et le 2-ethylhexyl dimethoxy benzylidene dioxoimidazolidine propionate.

Selon un aspect particulier, l'invention a pour objet une émulsion huile-dans-eau (E) telle que définie à la revendication 8.

Selon cet aspect particulier, ledit mélange (M₁) est plus particulièrement un mélange d'hydrocarbures saturés commercialisé sous le nom de marque Emogreen™L15 comprenant pour 100% de sa masse :

i) 3,7% d'alcanes linéaires comportant de quinze à dix-neuf atomes de carbone,
ii) 96% d'iso-alcanes comportant de quinze à dix-neuf atomes de carbone, et
iii) 0,3% de cyclo-alcanes comportant de quinze à dix-neuf atomes de carbone.

Selon un autre aspect plus particulier, l'invention a pour objet une émulsion huile-dans-eau (E) telle que définie à la revendication 10.

Selon cet aspect particulier, le mélange (M₁) est plus particulièrement un mélange d'hydrocarbures saturés commercialisé sous le nom de marque Emosmart™L19, comprenant pour 100% de sa masse :

i) 13,20% massique d'alcanes linéaires comportant de 15 à 19 atomes de carbone,
ii) 55,00% massique d'iso-alcanes comportant de 15 à 19 atomes de carbone, et
iii) 31,80% de cyclo-alcanes comportant de 15 à 19 atomes de carbone.

L'émulsion (E) telle que définie précédemment peut comprendre un ou plusieurs adjuvants tels que :

- Des agents épaississants ou gélifiants, par exemple es polymères de type polyélectrolytes, linéaires ou branchés ou réticulés, comme l'homopolymère de l'acide acrylique partiellement ou totalement salifié, l'homopolymère de l'acide méthacrylique partiellement ou totalement salifié, l'homopolymère de l'acide 2-méthyl-[(1-oxo-2-propényl)amino]-1-propanesulfonique (AMPS) partiellement ou totalement salifié, les copolymères de l'acide acrylique et de l'AMPS, les copolymères de l'acrylamide et de l'AMPS, les copolymères de la vinylpyrolidone et de l'AMPS, les copolymères de l'AMPS et de l'acrylate de (2-hydroxyéthyle), les copolymères de l'AMPS et du méthacrylate de (2-hydroxyéthyle), les copolymères de l'AMPS et de l'hydroxyéthylacrylamide, les copolymères de l'AMPS et du N,N-diméthyl acrylamide, les copolymères de l'AMPS et du tris(hydroxy- méthyl)acrylamido méthane (THAM), les copolymères de l'acide acrylique ou méthacrylique et de l'acrylate de (2-hydroxy éthyle), les copolymères de l'acide acrylique ou méthacrylique et du méthacrylate de (2-hydroxy éthyle), les copolymères de l'acide acrylique ou méthacrylique et de l'hydroxyéthylacrylamide, les copolymères de l'acide acrylique ou méthacrylique et du THAM, les copolymères de l'acide acrylique ou méthacrylique et du N,N-diméthyl acrylamide, les terpolymères de l'acide acrylique ou méthacrylique, de l'AMPS et de l'acrylate de (2-hydroxy éthyle), les terpolymères de l'acide acrylique ou méthacrylique, de l'AMPS et du méthacrylate de (2-hydroxy éthyle), les terpolymères de l'acide acrylique ou méthacrylique, de l'AMPS et du THAM, les terpolymères de l'acide acrylique ou méthacrylique, de l'AMPS et du N,N-diméthyl acrylamide, les terpolymères de l'acide acrylique ou méthacrylique, de l'AMPS et de l'acrylamide, les copolymères de l'acide acrylique ou de l'acide méthacrylique et d'acrylates d'alkyle dont la chaîne carbonée comprend entre quatre et trente atomes de carbone et plus particulièrement entre dix et trente atomes de carbone, les copolymères de l'AMPS et d'acrylates d'alkyle dont la chaîne carbonée comprend entre quatre et trente atomes de carbone et plus particulièrement entre dix et trente atomes de carbone, les terpolymères linéaire, branché ou réticulé d'au moins un monomère possédant une fonction acide fort, libre, partiellement salifiée ou totalement salifiée, avec au moins un monomère neutre, et au moins un monomère de formule (VIII) :

$$CH_2=C(R'_3)-C(=O)-[CH_2-CH_2-O]_n-R'_4 \qquad (VIII)$$

dans laquelle $R'_3$ représente un atome d'hydrogène ou un radical méthyle, $R'_4$ représente un radical alkyle linéaire ou ramifié comportant de huit à trente atomes de carbone et n représente un nombre supérieur ou égal à un et inférieur ou égal à cinquante; Les polymères de type polyélectrolytes, linéaires ou branchés ou réticulés que l'on peut associer à l'émulsion huile-dans-eau objet de la présente invention, peuvent se présenter sous la forme d'une solution, d'une suspension aqueuse, d'une émulsion eau-dans-huile, d'une émulsion huile-dans-eau, d'une poudre, par exemple les produits commercialisés sous les appellations SIMULGEL™ EG, SIMULGEL™EPG, SEPIGEL™ 305, SIMULGEL™ 600, SIMULGEL™ NS, SIMULGEL™ INS 100, SIMULGEL™ FL, SIMULGEL™ A, SIMULGEL™ SMS 88, SEPINOV™EMT 10, SEPIPLUS™400, SEPIPLUS™265, SEPIPLUS™S, SEPIMAX™Zen, ARISTOFLEX™AVC, ARISTOFLEX™AVS, NOVEMER™EC-1, NOVEMER™EC 2, ARISTOFLEX™HMB, COSMEDIA™SP, FLOCARE™ET 25, FLOCARE™ET 75, FLOCARE™ET 26, FLOCARE™ET 30, FLOCARE™ET 58, FLOCARE™PSD 30, VISCOLAM™AT 64, VISCOLAM™AT 100; les polysaccharides constitués uniquement d'oses, comme les glucanes ou homopolymères du glucose, les glucomannoglucanes, les xyloglycanes, les galactomannanes dont le degré de substitution (DS) des unités de D-galactose sur la chaîne principale de D-mannose est compris entre 0 et 1, et plus particulièrement entre 1 et 0,25, comme les galactomannanes provenant de la gomme de cassia (DS = 1/5), de la gomme de caroube (DS = 1/4), de la gomme de tara (DS = 1/3), de la gomme de guar (DS = 1/2), de la gomme de fenugrec (DS = 1 ); les polysaccharides constitués de dérivés d'oses, comme les galactanes sulfatés et plus particulièrement les carraghénanes et l'agar, les uronanes et plus particulièrement les algines, les alginates et les pectines, les hétéropolymères d'oses et d'acides uroniques et plus particulièrement la gomme xanthane, la gomme gellane, les exsudats de gomme de arabique et de gomme de karaya, les glucosaminoglycanes; la cellulose, les dérivés de cellulose comme la méthyl-cellulose, l'éthyl-cellulose, l'hydroxypropyl cellulose, les silicates, l'amidon, les dérivés hydrophiles de l'amidon, les polyuréthanes;

- Des composés filmogènes,
- Des agents hydrotropes,
- Des agents plastifiants,
- Des agents opacifiants et/ou nacrants, tels que les palmitate, stéarate ou les hydroxy-stéarate de sodium ou de magnésium, les monostéarates ou distéarate d'éthylène ou de polyéthylène glycol, les alcools gras, les homopolymères et copolymères de styrène tels que le styrène acrylate copolymère commercialisé sous l'appellation MONTOPOL™ OP1 par la société SEPPIC.
- Des agents de texture tels que la lauroyl lysine commercialisée sous l'appellation AMINOHOPE™LL par la société AJINOMOTO, l'octenyl starch succinate commercialise sous l'appellation DRYFLO™ par la société NATIONAL STARCH, le myristyl polyglucoside commercialisé par SEPPIC sous l'appellation MONTANOV™ 14, les fibres de cellulose, les fibres de coton, les fibres de chitosane, le talc, la sericite, le mica;
- Des agents surgraissants,
- Des agents séquestrants,
- Des agents chélatants,
- Des agents tensioactifs non-ioniques tels que les dérivés éthoxylés d'alcools gras comportant de 8 à 12 atomes de carbone, les dérivés éthoxylés d'acides gras comportant de 8 à 12 atomes de carbone; les dérivés éthoxylés d'esters gras comportant de 8 à 12 atomes de carbone; les dérivés éthoxylés de monoglycérides comportant de 8 à 12 atomes de carbone; les alkylpolyglycosides de formule (II):

$$R_2\text{-O-}(S)_y\text{-H} \qquad (II)$$

dans laquelle y représente un nombre décimal compris entre 1 et 5, S représente le reste d'un sucre réducteur et $R_2$ représente un radical alkyle linéaire ou ramifié, saturé ou insaturé, ayant de 5 à 16 atomes de carbone, de préférence de 8 à 14 atomes de carbone, ou un mélange de composés de formule (II), par exemple le caprylyl capryl glucosides, commercialisé notamment sous le nom de marque ORAMIX™CG 110, le décylglucoside, commercialisé notamment sous le nom de marque ORAMIX™NS 10;
- Des agents antioxydants, tels que l'EDTA et ses sels, l'acide citrique, l'acide tartrique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE GL 47S (nom INCI: Tetrasodium Glutamate Diacetate);
- Des parfums,
- Des agents conservateurs,
- Des agents conditionneurs,
- Des principes actifs destinés à apporter une action traitante vis à vis de la peau ou des cheveux, tels que les vitamines et leurs dérivés, notamment leurs esters, tels que le rétinol (vitamine A) et ses esters (palmitate de rétinyle par exemple), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés); les composés montrant une action apaisante notamment le SEPICALM™ S, l'allantoïne et le bisabolol; les agents anti-inflammatoires; les composés montrant une action hydratante comme l'urée, les hydroxyurées, le glycérolglucoside, le diglycérolglucoside, les polyglycérylglucosides; les extraits végétaux riches en polyphénols comme les extraits de raisin, les extraits de pin, les extraits de vin, les extraits d'olives; les composés montrant une action amincissante ou lipolytique comme la caféine ou ses dérivés, l'ADIPOSLIM™, l'ADIPOLESS™, la fucoxanthine; les protéines N-acylées; les peptides N-acylés comme le MATRIXIL™; les acides aminés N-acylés; les hydrolysâts partiels de protéines N-acylés; les acides aminés; les peptides; les hydrolysâts totaux de protéines; les extraits de soja, par exemple la Raffermine™; les extraits de blé par exemple la TENSINE™ ou la GLIADINE™; les extraits végétaux, tels que les extraits végétaux riches en tanins, les extraits végétaux riches en isoflavones ou les extraits végétaux riches en terpènes; les extraits d'algues d'eau douce ou marines; les extraits de plantes marines; les extraits marins en général comme les coraux; les cires essentielles; les extraits bactériens; les céramides; les phospholipides; les composés montrant une action antimicrobienne ou une action purifiante, comme le LIPACIDE™ C8G, le LIPACIDE™ UG, le SEPICONTROL™ A5; l'OCTOPIROX™ ou le SENSIVA™ SC50; les composés montrant une propriété énergisante ou stimulante comme le PHYSIOGENYL™, le panthénol et ses dérivés comme le SEPICAP™ MP; les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™; le SURVICODE™; les actifs anti-photo vieillissement; les actifs protecteurs de l'intégrité de la jonction dermo-épidermique; les actifs augmentant la synthèse des composants de la matrice extracellulaire comme le collagène, les élastines, les glycosaminoglycanes; les actifs agissant favorablement sur la communication cellulaire chimique comme les cytokines ou physiques comme les intégrines; les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés); les actifs améliorant la microcirculation cutanée, par exemple les veinotoniques; les actifs drainants; les actifs à visée décongestionnante comme les extraits de ginko biloba, de lierre, de marron d'inde, de bambou, de ruscus, de petit houx, de centalla asiatica, de fucus, de romarin, de saule; les agents de bronzage ou de brunissement de la peau, comme par exemple la dihydroxyacétone (DHA), l'érythrulose, l'aldéhyde mésotartrique, le glutaraldéhyde, le glycéraldéhyde, l'alloxane, la ninhydrine, les extraits végétaux comme par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971 683;; les agents connus pour leur action de facilitation et/ou d'accélération du bronzage et/ou du brunissement de la peau humaine, et/ou pour leur action de coloration de la peau humaine, comme par exemple les caraténoïdes ( et plus particulièrement le beta carotène et le gamma carotène), le produit commercialisé sous le nom de marque « Carrot oil » (Nom INCI: Daucus Carota, helianthus annuus Sunflower oil) par la société Provital, qui contient des caroténoïdes, de la vitamine E et de la vitamine K; la tyrosine et/ou ses dérivés, connus pour leur effet sur l'accélération du bronzage de la peau humaine en association avec une exposition aux rayonnements ultra-violets, comme par exemple le produit commercialisé sous le nom de marque « SunTan Accelerator™ » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze™ (nom INCI: Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI: butylene glycol and Acetyl Tyrosine and hydrolyzed vegetable protein and Adenosine triphosphate) par la société UNIPEX, le produit commercialisé sous le nom de marque « Try-Excell™ » (nom INCI: Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze™ » (nom INCI: hydrolyzed wheat protein and acetyl tyrosine and copper gluconate) par la société Alban Muller, le produit commercialisé sous le nom de marque Tyrostan™ (nom INCI: potassium caproyl tyrosine) par la société Synerga, le produit commercialisé sous le nom de marque Tyrosinol (nom INCI: Sorbitan Isostearate, glyceryl oleate, caproyl Tyrosine) par la société Synerga, le produit commercialisé sous le nom de marque InstaBronze™ (nom INCI: Dihydroxyacetone and acetyl tyrosine and copper gluconate) commercialisé par la société Alban Muller, le produit commercialisé sous le nom de marque Tyrosilane (nom INCI: méthylsilanol and acétyl tyrosine) par la société Exymol; les peptides connus pour leur effet d'activation de la mélanogénèse comme par exemple le produit commercialisé sous le nom de marque Bronzing SF Peptide powder (nom INCI: Dextran and Octapeptide-5) par la société Infinitec Activos, le produit commercialisé sous le nom de marque Melitane (nom INCI: Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions™ (nom INCI: Butylene glycol, Palmitoyl Tripeptide-40) par la société LIPOTEC, les sucres et les dérivés de sucres comme par exemple le produit commercialisé sous le nom de marque Tanositol™ (nom INCI: inositol) par la société Provital, le produit commercialisé sous le nom de marque Thalitan™ (ou Phycosaccharide™ AG) par la société CODIF international (nom INCI: Aqua and hydrolyzed algin (Laminaria Digitata) and magnesium sulfate and manganese sulfate) contenant un oligosaccharide d'origine marine (acide guluronique et acide mannuronique chélatés avec les ions magnésium et manganèse), le produit commercialisé sous le nom de marque Melactiva™ (nom INCI: Maltodextrin, Mucuna Pruriens Seed extract) par la société Alban Muller, les composés riches en flavonoïdes comme par exemple le produit commercialisé sous le nom de marque « Biotanning » (nom INCI: Hydrolyzed citrus Aurantium dulcis fruit extract) par la société Silab et connu pour être riche en flavonoides de citron (de type hespéridines);

- Des charges minérales ou des pigments, tels que le dioxyde de titane, les oxydes de fer marrons, les oxydes de fer jaune, les oxydes de fer noir, ou les oxydes de fer rouges ou encore les pigments nacrés blancs ou colorés tels que les Mica-Titane.
- Des particules procurant un effet visuel ou destinées à l'encapsulation d'actifs,
- Des particules exfoliantes,
- Des azurants optiques,
- Des agents répulsifs des insectes.

L'émulsion huile-dans-eau (E) telle que définie précédemment peut être conditionnée dans un flacon, dans un dispositif de type "flacon" pompe, sous forme pressurisées dans un dispositif aérosol, dans un dispositif muni d'une paroi ajourée comme une grille ou dans un dispositif muni d'un applicateur à billes (dit "roll-on").

L'émulsion de type huile-dans-eau à usage topique (E) telle que définie précédemment peut être utilisée pour la protection de la peau humaine contre les effets inesthétiques de l'exposition aux rayonnements ultra-violets du soleil, et plus particulièrement contre les rougeurs.

L'invention a enfin pour objet une émulsion une émulsion de type huile-dans-eau (E) telle que définie à la revendication 12.

Les exemples suivants illustrent l'invention sans toutefois la limiter.

**Préparation d'émulsions huile-dans-eau selon l'invention, comprenant un mélange** $(M_1)$, **et d'émulsions huile-dans-eau comparatives.**

On prépare quatre émulsions huile-dans-eau selon l'invention, notées $(E_1)$ à $(E_4)$, dont les proportions massiques de leurs constituants sont indiquées dans le tableau 1, et six émulsions huile-dans-eau comparatives notées $(F_1)$ à $(F_5)$ dont les proportions massiques de leurs constituants sont indiquées dans le tableau 2 ci-dessous. Le procédé de préparation commun pour les émulsions huile-dans-eau selon l'invention et pour les émulsions huile-dans-eau comparatives, est le suivant :

- On verse dans un bécher, à une température de 20°C, le mélange huileux à tester, puis le cas échéant on disperse progressivement une éventuelle seconde huile sous agitation mécanique à 80 tours/minute ;
- On y ajoute ensuite, la quantité requise de l'agent épaississant SEPINOV™EMT 10 sous agitation mécanique à 80 tours/minute et à 20°C;
- On prépare par mélange dans un bécher, à une température de 20°C, la phase aqueuse comprenant l'eau et l'Oramix™NS10,
- Le mélange alors obtenu est refroidi sous agitation de type défloculeuse à 1500 tours par minute pendant vingt minutes, puis vidangé pour obtenir l'émulsion huile-dans-eau souhaitée.

**Tableau 1**

| | Emulsion | | | |
|---|---|---|---|---|
| | $(E_1)$ | $(E_2)$ | $(E_3)$ | $(E_4)$ |
| **Phase grasse** | | | | |
| Sepinov™EMT10[(1)] | 2% | 2% | 2% | 2% |
| Emosmart™L19[(2] | 15% | 0% | 12% | 3% |
| Emogreen™L15[(3)] | 0% | 15% | 3% | 12% |
| **Phase aqueuse** | | | | |
| Eau | Qs. 100% | Qs. 100% | Qs. 100% | Qs. 100% |
| Oramix™NS 10[(4)] | 0,3% | 0,3% | 0,3% | 0,3% |
| (1) (Sepinov™EMT10): Agent épaississant (nom INCI: hydroxyethyl acrylate / acryloyldimethyltaurate acrylate copolymer), (2) (Emosmart™L19): Mélange d'hydrocarbures saturés cycliques ou acycliques, linéaires ou ramifiés comprenant pour 100% de sa masse: i) 13,20% massique d'alcanes linéaires comportant de 15 à 19 atomes de carbone, ii) 55,00% massique d'iso-alcanes comportant de 15 à 19 atomes de carbone, iii) 31,80% de cyclo-alcanes comportant de 15 à 19 atomes de carbone ; (3) (Emogreen™L15) : Mélange comprenant pour 100% de sa masse : i) 3,7% d'alcanes linéaires comportant de 15 à 19 atomes de carbone, ii) 96% % d'iso-alcanes comportant de 15 à 19 atomes de carbone, iii) 0,3 % de cyclo-alcanes comportant de 15 à 19 atomes de carbone ; (4) (Oramix™NS10): agent moussant solubilisant (nom INCI capryloyl/capryl glucoside). |

**Tableau 2**

| | Emulsion | | | | |
|---|---|---|---|---|---|
| | $(F_1)$ | $(F_2)$ | $(F_3)$ | $(F_4)$ | $(F_5)$ |
| **Phase grasse** | | | | | |
| Sepinov™EMT10 | 2% | 2% | 2% | 2% | 2% |
| Isohexadécane | 15% | 0% | 0% | 0% | 0% |
| DC 345[5] | 0% | 15% | 0% | 0% | 0% |
| DC 245[6] | 0% | 0% | 15% | 0% | 0% |
| Emosmart™L15[7] | 0% | 0% | 0% | 15% | 0% |
| Emosmart™V21[8] | 0% | 0% | 0% | 0% | 15% |
| **Phase aqueuse** | | | | | |
| Eau | Qs. 100% | Qs. 100% | Qs. 100% | Qs. 100% | Qs. 100% |
| Oramix™NS10 | 0,3% | 0,3% | 0,3% | 0,3% | 0,3% |

(5) (DC 345): Agent émollient utilisé en cosmétique pour les propriétés sensorielles qu'il procure et plus particulièrement la facilité d'étalement qu'il confère à l'émulsion le contenant (nom INCI cyclopentasiloxane & cyclohexasiloxane);

(6) (DC 245): Agent émollient utilisé en cosmétique pour les propriétés sensorielles qu'il procure et plus particulièrement la facilité d'étalement qu'il confère à l'émulsion le contenant; (nom INCI nom INCI cyclopentasiloxane) (7) (Emosmart™L15): Mélange comprenant pour 100% de sa masse:

i) 9,26% massique d'alcanes linéaires comportant de 13 à 15 atomes de carbone, ii) 39,06 % massique d'iso-alcanes comportant de 13 à 15 atomes de carbone, iii) 51,68 % de cyclo-alcanes comportant de 13 à 15 atomes de carbone;

(8) (Emosmart™V21): Mélange comprenant pour 100% de sa masse:

i) 15,99 % massique d'alcanes linéaires comportant de 18 à 21 atomes de carbone, ii) 59,90% massique d'iso-alcanes comportant de 18 à 21 atomes de carbone, iii) 24,11 % de cyclo-alcanes comportant de 18 à 21 atomes de carbone.

**Evaluation des propriétés d'étalement d'une émulsion huile-dans-eau selon l'invention et d'émulsions huile-dans-eau comparatives**

### Principe de la méthode

Les propriétés d'étalement d'une émulsion huile-dans-eau sont évaluées par la variation d'une moyenne de mesures de la valeur du coefficient de friction de ladite émulsion, réalisée à l'aide d'un rhéomètre de type DHR2 (de la société Texas Instrument), pour différentes vitesses d'étalement.

### Principe de la mesure

Il s'agit de caractériser chaque émulsion testée par une valeur moyenne de 3 valeurs mesurées du coefficient de friction, en force normale, pour une vélocité de 1 radian.s$^{-1}$ et de 4 radian.s$^{-1}$, puis de calculer le pourcentage d'évolution pour la moyenne obtenue à chacune des deux vitesses.

### Matériel et appareillage

Les mesures sont réalisées par l'intermédiaire d'un rhéomètre DHR2 (TA Instrument) équipé de l'accessoire de type « Tribo Ring on Plate » sur lequel est posé une surface de plexiglas sur laquelle est déposée l'émulsion à tester.

### Mode opératoire

- On dépose, à l'aide d'un étaleur calibré, une quantité de l'émulsion à tester telle qu'elle forme une épaisseur de 90 micromètres sur le plan.

- Après dépôt de l'échantillon, on met son épaisseur calibrée en contact avec la géométrie « Tribo Ring on Plate », et on applique une force normale de 2 N et un gradient de vélocité fixe pouvant être réglé à une valeur comprise entre 0,01 et 15 radian.s$^{-1}$.
- On mesure la force normale maximale de friction que subit le plan Peltier lors de la rotation de la géométrie.

<u>Expression des résultats</u>

Pour chaque émulsion testée, et pour chaque mesure effectuée de la valeur de la force normale maximale de friction, on calcule le coefficient de friction (Cf) comme suit :

$$Cf = (valeur\ force\ normale\ maximale\ de\ friction)\ /\ (valeur\ force\ normale\ appliquée)$$

Cf = (valeur force normale maximale de friction) / (valeur force normale appliquée)

Pour chaque émulsion testée, on considère les valeurs obtenues lors de 3 mesures statistiquement significatives, pour une vélocité fixée à 1 radian.s$^{-1}$ et pour une vélocité fixée à 4 radian.s$^{-1}$, et pour chacune des émulsions testées, on calcule la valeur moyenne des valeurs des coefficients de friction ainsi expérimentalement obtenues notée $Cf_{m1}$ pour une vélocité fixée à 1 radian.s$^{-1}$ et $Cf_{m4}$ pour une vélocité fixée à 4 radian.s$^{-1}$.

On calcule alors la variation (notée ΔCf) entre la valeur moyenne du coefficient de friction obtenue pour une vélocité de 1 radian.s$^{-1}$ et la valeur moyenne du coefficient de friction obtenue pour une vélocité de pour une vélocité fixée à 4 radian.s$^{-1}$ comme suit :

$$\Delta_{Cf} = (Cf_{m1} - Cf_{m4})\ /\ (Cf_{m1})\ x\ 100$$

<u>Résultats</u>

Les résultats obtenus sont consignés dans le tableau 3 ci-après.

**Tableau 3**

| | Emulsion | | | |
|---|---|---|---|---|
| | **(E$_1$)** | **(F$_1$)** | **(F$_2$)** | **(F$_3$)** |
| **Δ$_{Cf}$** | -6,8% | +18,2% | +17,5% | +14,5% |

<u>Analyse des Résultats</u>

Les résultats consignés dans le tableau 3 font apparaître clairement que la variation des moyennes des coefficients de friction, entre un étalement à une vélocité de 1 radian.s$^{-1}$ et une vélocité de 4 radian.s$^{-1}$ pour l'émulsion (E$_1$) selon l'invention est inférieure à une valeur de 10% (-6,8%) alors que les émulsions comparatives **(F$_2$)** et (F$_3$), comprenant des huiles de silicone connues pour conférer une amélioration de l'étalement aux émulsions qui la comprennent, montrent une variation du coefficient de friction supérieure à 15% entre un étalement à une vélocité de 1 radian.s$^{-1}$ et une vélocité de 4 radian.s$^{-1}$.

Ces résultats montrent ainsi une amélioration de la facilité d'étalement des émulsions selon l'invention par rapport à des émulsions comprenant des agents émollients connus pour procurer une facilité d'étalement aux émulsions le comprenant. La variation des moyennes des coefficients de friction, entre un étalement à une vélocité de 1 radian.s$^{-1}$ et une vélocité de 4 radian.s$^{-1}$ pour l'émulsion comparative (F$_1$), comprenant l'isohexadécane, soit une iso-paraffine comportant 16 atomes de carbone, montre une valeur de 18,2%, soit une moindre performance et une moindre facilité d'étalement pour les émulsions comprenant cette iso-paraffine comportant 16 atomes de carbone que pour les émulsions selon l'invention.

Enfin, la variation négative de la moyenne des coefficients de friction, entre une vélocité de 1 radian.s$^{-1}$ et une vélocité de 4 radian.s$^{-1}$, montre un bénéfice technique supplémentaire, à savoir l'obtention d'une plus faible valeur moyenne du coefficient de friction pour une faible vélocité, soit une possibilité d'étaler plus facilement sur la peau l'émulsion selon l'invention.

**Evaluation des propriétés sensorielles d'émulsions huile-dans-eau selon l'invention et d'émulsions huile-dans-eau comparatives**

Principe de la méthode

Treize panélistes dûment entraînés et habilités ont évalué les critères de "sensation de richesse" de "brillance de la peau" et de "brillance de l'émulsion" d'émulsions huile-dans-eau selon l'invention et d'émulsions huile-dans-eau comparatives, en prenant comme base de référence une émulsion connue par la personne du métier comme constituant une émulsion pour laquelle le critère de richesse est reconnu par l'ensemble des panélistes, ainsi qu'une référence de comparaison à surpasser pour réaliser le problème technique posé.

Mode opératoire

Le mode opératoire mis en œuvre comprend 5 étapes qui sont les suivantes :

- Etape 1 contrôle et évaluation de l'aspect et de l'odeur de l'émulsion huile-dans eau testée,
- Etape 2 : prise en main : évaluation de la facilité de préhension, et observation d'un éventuel aspect filant,
- Etape 3 : étalement sur la peau de l'émulsion testée par application circulaire à la surface de la peau, à une même vitesse pendant 10 tours, et recueil des sensations perçues à la fin du 10$^{ème}$ tour,
- Etape 4 : poursuite de l'étalement de l'émulsion testée sur la peau toujours par application du même mouvement circulaire jusqu'à observation de l'absence de film d'émulsion sur la peau et recueil des sensations perçues,
- Etape 5 : recueil des sensations perçues après 1 minute après la fin de l'étalement.

Ce mode opératoire est mis en œuvre à une température de 20°C.

Expression des résultats

Pour chaque émulsion testée, et pour chaque critère sensoriel évalué, chaque panéliste indique si ladite émulsion testée procure une sensation amélioré par rapport à l'émulsion de référence. L'ensemble des évaluations est collecté et les données sont traitée de façon statistique de façon à déterminer le caractère significatif d'une éventuelle différence, amélioration ou dégradation, entre la sensation perçue pour l'émulsion testée et l'émulsion de référence.

Résultats

Critère de "sensation de richesse"

- Emulsion de référence : émulsion $(F_4)$.

Les émulsions selon l'invention $(E_1)$ et $(E_3)$, ainsi que les émulsions comparatives **$(F_2)$** et $(F_5)$ sont évaluées selon le protocole défini ci-dessus et les résultats obtenus sont consignés dans le tableau 4 ci-dessous. L'amélioration de la sensation de richesse par rapport à $(F_4)$ est notée ">$(F_4)$" et la détérioration de la sensation de richesse par rapport à $(F_4)$ est notée "<$(F_4)$" à chaque moment du processus d'étalement.

**Tableau 4**

|  | Emulsion | | | |
|---|---|---|---|---|
|  | **$(E_1)$** | **$(E_3)$** | **$(F_2)$** | **$(F_5)$** |
| Etape 1: Evaluation visuelle | >$(F_4)$ | >$(F_4)$ | <$(F_4)$ | <$(F_4)$ |
| Etape 2: Evaluation à la préhension | >$(F_4)$ | >$(F_4)$ | <$(F_4)$ | <$(F_4)$ |
| Etape 3: Evaluation après les 10 premiers tours | >$(F_4)$ | >$(F_4)$ | <$(F_4)$ | <$(F_4)$ |
| Etape 4: Evaluation après pénétration | >$(F_4)$ | >$(F_4)$ | <$(F_4)$ | <$(F_4)$ |
| Etape 5: Evaluation 1 minute après fin de l'étalement | >$(F_4)$ | >$(F_4)$ | <$(F_4)$ | <$(F_4)$ |

Critère de "brillance de la peau"

- Emulsion de référence : émulsion $(F_4)$;

Les émulsions selon l'invention $(E_1)$ et $(E_3)$, ainsi que les émulsions comparatives **$(F_2)$** et $(F_5)$ sont évaluées selon

le protocole défini ci-dessus et les résultats obtenus sont consignés dans le tableau 5 ci-dessous. L'augmentation de la brillance de la peau par rapport à $(F_4)$ est notée ">$(F_4)$" et sa diminution notée "<$(F_4)$".

**Tableau 5**

| | Emulsion | | | |
|---|---|---|---|---|
| | $(E_1)$ | $(E_3)$ | $(F_2)$ | $(F_5)$ |
| Etape 5 : Evaluation 1 minute après fin de l'étalement | <$(F_4)$ | <$(F_4)$ | >$(F_4)$ | >$(F_4)$ |

Critère de "brillance de l'émulsion"

Les émulsions selon l'invention $(E_1)$ et $(E_3)$, ainsi que les émulsions comparatives **$(F_2)$** et $(F_5)$ sont évaluées selon le protocole défini ci-dessus et les résultats obtenus sont consignés dans le tableau 5 ci-dessous. L'augmentation de la brillance de l'émulsion par rapport à $(F_4)$ est notée ">$(F_4)$" et sa diminution notée "<$(F_4)$".

**Tableau 6**

| | Emulsion | | | |
|---|---|---|---|---|
| | $(E_1)$ | $(E_3)$ | $(F_2)$ | $(F_5)$ |
| Etape 1: Evaluation visuelle | >$(F_4)$ | >$(F_4)$ | <$(F_4)$ | <$(F_4)$ |

Analyse des résultats

Les résultats obtenus montrent l'amélioration des propriétés sensorielles et esthétiques grâce au procédé selon l'invention.

**Formules illustratives**

**Émulsion solaire**

| Ingrédients | % (massique) |
|---|---|
| Polyacrylate Crosspolymer-6 (SEPIMAX™ ZEN) | 0.90% |
| Glycérine | 1.50% |
| Aqua | Qsp 100% |
| Easynov™ | 3.00% |
| EMOGREEN™L15 | 12.00% |
| Sepicide™ HB | 1.00% |
| Fragrance | 0.30% |
| Titanium Dioxide And Alumina And Stearic Acid | 10.00% |

**Fluide solaire haute protection UV**

| Ingrédients | % (massique) |
|---|---|
| Montanov™ 82 | 2.00% |
| C12-15 Alkyl Benzoate | 17.00% |
| Octocrylene | 6.00% |

(suite)

| Ingrédients | % (massique) |
|---|---|
| Ethylhexyl Methoxycinnamate | 6.00% |
| Bis-Ethylhexylphenol Methoxyphenyl triazine | 3.00% |
| Emogreen ™L19 | 3.00% |
| Tocopherol | 0.05% |
| Polyacrylate Crosspolymer-6 (SEPIMAX™ ZEN) | 0.25% |
| Cyclopentasiloxane | 5.00% |
| Titanium Dioxide And Isohexadecane And Triethylhexanoin And Aluminium Stearate And Alumina And Polyhydroxystearic Acid | 5.40% |
| Aqua | Qsp 100% |
| Methylene Bis-Benzotriazolyl Tetramethylbutylphenol | 10.00% |
| Citric Acid | Qs pH 5.5 |
| Aquaxyl™ | 3.00% |
| Phenoxyethanol Ethylhexylglycerin | 1.00% |
| Fragrance | 0.20% |

**Fluide solaire haute protection UV**

| Ingrédients | % (massique) |
|---|---|
| Montanov™ 82 | 2.00% |
| C12-15 Alkyl Benzoate | 17.00% |
| Octocrylene | 6.00% |
| Ethylhexyl Methoxycinnamate | 6.00% |
| 2-ethylhexyl dimethoxybenzylidene dioxoimidazolidine propionate | 3.00% |
| Emogreen™L15 | 3.00% |
| Tocopherol | 0.05% |
| Polyacrylate Crosspolymer-6 (SEPIMAX™ ZEN) | 0.25% |
| Cyclopentasiloxane | 5.00% |
| Titanium Dioxide And Isohexadecane And Triethylhexanoin And Aluminium Stearate And Alumina And Polyhydroxystearic Acid | 5.40% |
| Aqua | Qsp 100% |
| Methylene Bis-Benzotriazolyl Tetramethylbutylphenol | 10.00% |
| Citric Acid | Qs pH 5.5 |
| Aquaxyl™ | 3.00% |
| Phenoxyethanol Ethylhexylglycerin | 1.00% |

(suite)

| Ingrédients | % (massique) |
|---|---|
| Fragrance | 0.20% |

SEPIMAX™ Zen (Polyacrylate Crosspolymer-6) est un polymère anionique épaississant et stabilisant, commercialisé par la société SEPPIC EASYNOV™ (Octyldodecanol & Octydodecyl Xyloside & PEG-30 Dipolyhydroxystearate) est un agent émulsionnant de type eau-dans-huile, commercialisé par la société SEPPIC SOLAGUM™ AX (Acacia Senegal Gum and Xanthan Gum) est un polymère épaississant et stabilisant d'origine naturelle, commercialisé par la société SEPPIC.

Montanov™ 82 (Cetearyl Alcohol & Cocoglucoside) est un agent émulsionnant de type huile-dans-eau, commercialisé par la société SEPPIC AQUAXYL™ (Xylitylglucoside and Anhydroxylitol and Xylitol) est un actif hydratant commercialisée par la société SEPPIC.

SEPICIDE™ HB (Phenoxyethanol & Methylparaben & Ethylparaben & Propylparaben & Butylparaben) est une composition conservatrice commercialisée par la société SEPPIC.

Concentré de parfum « Citrus Waterfall » est commercialisé par la société Mane Montanov™ 202 (Arachidyl Alcohol (and) Behenyl Alcohol. (and) Arachidyl Glucoside) est un agent émulsionnant de type huile-dans-eau, commercialisé par la société SEPPIC

**Revendications**

1. Procédé pour améliorer les propriétés sensorielles et/ou esthétiques d'une émulsion (E$_0$) de type huile-dans-eau, lesdites propriétés sensorielles étant les propriétés d'étalement, les propriétés de consistance et de richesse, de ladite émulsion à usage topique (E$_0$), **caractérisé en ce que** l'on y incorpore une quantité efficace d'un mélange (M$_1$) d'hydrocarbures saturés cycliques ou acycliques, linéaires ou ramifiés comprenant pour 100% de sa masse soit :

   - Une proportion massique en alcanes ramifiés supérieure ou égale à 80% et inférieure ou égale à 100% ;
   - Une proportion massique en alcanes linéaires supérieure ou égale à 0% et inférieure ou égale à 15% ;
   - Une proportion massique en cyclo-alcanes supérieure ou égale à 0% et inférieure ou égale à 5% et dans lequel de 95% massique à 100% massique desdits hydrocarbures saturés cycliques ou acycliques, linéaires ou ramifiés, comportent de quinze à dix-neuf atomes de carbone, et jusqu'à 5% massique desdits hydrocarbures saturés cycliques ou acycliques, linéaires ou ramifiés, comportent moins de quinze atomes de carbone ou plus de dix-neuf atomes de carbone

   soit :

   - Une proportion massique en alcanes ramifiés supérieure ou égale à 40% et inférieure ou égale à 100%,
   - Une proportion massique en alcanes linéaires supérieure ou égale à 0% et inférieure ou égale à 20%,
   - Une proportion massique en cyclo-alcanes supérieure ou égale à 0% et inférieure ou égale à 40% et dans lequel 100% massique desdits hydrocarbures saturés cycliques ou acycliques, linéaires ou ramifiés, comportent de quinze à dix-neuf atomes de carbone ;

   **et en ce que** ladite émulsion (E$_0$) est exempte de beurre de karité.

2. Procédé tel que défini à la revendication 1, dans lequel le mélange (M$_1$) mis en œuvre comprend pour 100% de sa masse :

   - Une proportion massique en alcanes ramifiés supérieure ou égale à 90% et inférieure ou égale à 100%,
   - Une proportion massique en alcanes linéaires supérieure ou égale à 0% et inférieure ou égale à 10%,
   - Une proportion massique en cycloalcanes supérieure ou égale à 0% et inférieure ou égale à 1%

   et dans lequel de 95% massique à 100% massique desdits hydrocarbures saturés cycliques ou acycliques, linéaires ou ramifiés, comportent de quinze à dix-neuf atomes de carbone, et jusqu'à 5% massique desdits hydrocarbures saturés cycliques ou acycliques, linéaires ou ramifiés, comportent moins de quinze atomes de carbone ou plus de dix-neuf atomes de carbone.

3. Procédé tel que défini à la revendication 1, dans lequel le mélange ($M_1$) mis en œuvre comprend pour 100% de sa masse :

- Une proportion massique en alcanes ramifiés supérieure ou égale à 50% et inférieure ou égale à 100%,
- Une proportion massique en alcanes linéaires supérieure ou égale à 0% et inférieure ou égale à 15%,
- Une proportion massique en cycloalcanes supérieure ou égale à 0% et inférieure ou égale à 35%,

et dans lequel 100% massique desdits hydrocarbures saturés cycliques ou acycliques, linéaires ou ramifiés, comportent de quinze à dix-neuf atomes de carbone.

4. Procédé tel que défini à la revendication 2, dans lequel le mélange ($M_1$) mis en œuvre comprend pour 100% de sa masse :

- Une proportion massique en alcanes linéaires égale à 3,7%,
- Une proportion massique en iso-alcanes égale à 96%, et
- Une proportion massique en cyclo-alcanes égale à 0,3%.

5. Procédé tel que défini à la revendication 3, dans lequel le mélange ($M_1$) mis en œuvre comprend pour 100% de sa masse :

- Une proportion massique en alcanes linéaires égale à 13,20%,
- Une proportion massique en iso-alcanes égale à 55,00% et
- Une proportion massique en cyclo-alcanes égale à 31,80%.

6. Emulsion à usage topique de type huile-dans-eau (E), **caractérisée en ce qu'**elle comprend pour 100% de sa masse :

- De 50% à 90% massique, d'une phase aqueuse (A) cosmétiquement acceptable,
- De 10% à 50% massique, d'une phase grasse (G) comprenant pour 100% de sa masse :

- De 10% à 50% massique, plus particulièrement de 15% à 40% massique d'un mélange ($M_1$) d'hydrocarbures saturés cycliques ou acycliques, linéaires ou ramifiés, ledit mélange ($M_1$) comprenant pour 100% de sa masse soit :

- Une proportion massique en alcanes ramifiés supérieure ou égale à 80% et inférieure ou égale à 100%,
- Une proportion massique en alcanes linéaires supérieure ou égale à 0% et inférieure ou égale à 15%,
- Une proportion massique en cyclo-alcanes supérieure ou égale à 0% et inférieure ou égale à 5%,

et dans lequel de 95% massique à 100% massique desdits hydrocarbures saturés cycliques ou acycliques, linéaires ou ramifiés, comportent de quinze à dix-neuf atomes de carbone, et jusqu'à 5% massique desdits hydrocarbures saturés cycliques ou acycliques, linéaires ou ramifiés, comportent moins de quinze atomes de carbone ou plus de dix-neuf atomes de carbone ;
soit :

- Une proportion massique en alcanes ramifiés supérieure ou égale à 40% et inférieure ou égale à 100%,
- Une proportion massique en alcanes linéaires supérieure ou égale à 0% et inférieure ou égale à 20%,
- Une proportion massique en cyclo-alcanes supérieure ou égale à 0% et inférieure ou égale à 40% et dans lequel 100% massique desdits hydrocarbures saturés cycliques ou acycliques, linéaires ou ramifiés, comportent de quinze à dix-neuf atomes de carbone,
- De 0,5% à 15% massique, d'au moins un agent tensioactif de type huile-dans-eau,
- De 5% à 30% massique, d'au moins un agent de protection contre les rayonnements ultra-violets du soleil,
- De 0% à 80% massique, d'au moins une huile et/ou une cire, étant entendu qu'une telle au moins une huile et/ou une telle cire, ne répond pas à la définition du mélange ($M_1$) ;

**et en ce qu'**elle est exempte de beurre de karité.

7. Emulsion huile-dans-eau à usage topique (E) telle que définie à la revendication 6, **caractérisée en ce que** l'agent de protection contre les rayonnements ultra-violets du soleil est choisi parmi les éléments du groupe constitué par le dioxyde de titane, la 2,4-dihydroxy benzophénone, le 2-(4-diethylamino-2-hydroxybenzoyl) benzoic acid hexyl ester, le 2,4-bis { [4-(2-ethylhexyloxy)-2-hydroxy] phenyl} -6-(4-methoxyphenyl)-1,3,5-triazine, le 2,4,6-tris[4-(2-ethylhexyloxy carbonyl) anilino]-1,3,5-triazine et le 2-ethylhexyl dimethoxy benzylidene dioxoimidazolidine propionate.

8. Emulsion huile-dans-eau (E) telle que définie à l'une quelconque des revendications 6 ou 7, **caractérisée en ce que** ledit mélange ($M_1$) comprend pour 100% de sa masse :

- Une proportion massique en alcanes ramifiés supérieure ou égale à 90% et inférieure ou égale à 100%,
- Une proportion massique en alcanes linéaires supérieure ou égale à 0% et inférieure ou égale à 10%,
- Une proportion massique en cycloalcanes supérieure ou égale à 0% et inférieure ou égale à 1%,

dans lequel de 95% massique à 100% massique desdits hydrocarbures saturés cycliques ou acycliques, linéaires ou ramifiés, comportent de quinze à dix-neuf atomes de carbone et jusqu'à 5% massique desdits hydrocarbures saturés cycliques ou acycliques, linéaires ou ramifiés, comportent moins de quinze atomes de carbone ou plus de dix-neuf atomes de carbone.

9. Emulsion huile-dans-eau (E) telle que définie à la revendication 8, **caractérisée en ce que** ledit mélange ($M_1$) comprend pour 100% de sa masse :

- Une proportion massique en alcanes linéaires égale à 3,7% ;
- Une proportion massique en iso-alcanes égale à 96% ;
- Une proportion massique en cyclo-alcanes égale à 0,3%.

10. Emulsion à usage topique de type huile-dans-eau (E) telle que définie à l'une quelconque des revendications 6 ou 7 **caractérisé en ce que** ledit mélange ($M_1$) comprend pour 100% de sa masse :

- Une proportion massique en alcanes ramifiés supérieure ou égale à 50%,
- Une proportion massique en alcanes linéaires supérieure ou égale à 0% et inférieure ou égale à 15%,
- Une proportion massique en cycloalcanes supérieure ou égale à 0% et inférieure ou égale à 35%,

dans lequel 100% massique desdits hydrocarbures saturés cycliques ou acycliques, linéaires ou ramifiés, comportent de quinze à dix-neuf atomes de carbone.

11. Emulsion à usage topique de type huile-dans-eau (E) telle que définie à la revendication 10, **caractérisé en ce que** ledit mélange ($M_1$) comprend pour 100% de sa masse :

- Une proportion massique en alcanes linéaires égale à 13,20%,
- Une proportion massique en iso-alcanes égale à 55,00% et
- Une proportion massique en cyclo-alcanes supérieure ou égale à 31,80%.

12. Emulsion à usage topique de type huile-dans-eau (E) telle que définie à l'une quelconque des revendications 6 à 11, pour son utilisation dans une méthode de traitement thérapeutique destinée à prévenir et/ou traiter des maladies de la peau humaine liée à une exposition aux rayonnements ultra-violets du soleil, plus particulièrement les brûlures, les coups de soleil, les érythèmes, les cancers de la peau.

**Patentansprüche**

1. Verfahren zum Verbessern der sensorischen und/oder ästhetischen Eigenschaften einer Emulsion ($E_0$) des Öl-in-Wasser-Typs, wobei die sensorischen Eigenschaften die Ausbreitungseigenschaften, die Konsistenz- und Reichhaltigkeitseigenschaften der Emulsion für eine topische Verwendung ($E_0$) sind, **dadurch gekennzeichnet, dass** eine wirksame Menge einer Mischung ($M_1$) von gesättigten, cyclischen oder acyclischen, linearen oder verzweigten Kohlenwasserstoffen untergemischt werden, das heißt umfassend für 100 % ihrer Masse entweder:

- einen Massenanteil an verzweigten Alkanen, der größer als oder gleich 80 % und kleiner als oder gleich 100 % ist;
- einen Massenanteil an linearen Alkanen, der größer als oder gleich 0 % und kleiner als oder gleich 15 % ist;
- einen Massenanteil an Cycloalkanen, der größer als oder gleich 0 % und kleiner als oder gleich 5 % ist; und wobei von zu 95 Massen-% bis 100 Massen-% der gesättigten cyclischen oder acyclischen, linearen oder verzweigten, Kohlenwasserstoffe, von fünfzehn bis neunzehn Kohlenstoffatome aufweisen, und bis zu 5 Massen-% der gesättigten cyclischen oder acyclischen, linearen oder verzweigten, Kohlenwasserstoffe weniger als fünfzehn Kohlenstoffatome oder mehr als neunzehn Kohlenstoffatome aufweisen, oder:

- einen Massenanteil an verzweigten Alkanen, der größer als oder gleich 40 % und kleiner als oder gleich 100 % ist,
- einen Massenanteil an linearen Alkanen, der größer als oder gleich 0 % und kleiner als oder gleich 20 % ist,
- einen Massenanteil an Cycloalkanen, der größer als oder gleich 0 % und kleiner als oder gleich 40 % ist, und wobei zu 100 Massen-% der gesättigten cyclischen oder acyclischen, linearen oder verzweigten, Kohlenwasserstoffe von fünfzehn bis neunzehn Kohlenstoffatome aufweisen;
**und dadurch dass** die Emulsion ($E_0$) frei von Sheabutter ist.

2. Verfahren nach Anspruch 1, wobei die umgesetzte Mischung ($M_1$) zu 100 % ihrer Masse umfasst:

- einen Massenanteil an verzweigten Alkanen, der größer als oder gleich 90 % und kleiner als oder gleich 100 % ist,
- einen Massenanteil an linearen Alkanen, der größer als oder gleich 0 % und kleiner als oder gleich 10 % ist,
- einen Massenanteil an Cycloalkanen, der größer als oder gleich 0 % und kleiner als oder gleich 1 % ist;
und wobei von zu 95 Massen-% bis 100 Massen-% der gesättigten cyclischen oder acyclischen, linearen oder verzweigten, Kohlenwasserstoffe von fünfzehn bis neunzehn Kohlenstoffatome aufweisen, und bis zu 5 Massen-% der gesättigten cyclischen oder acyclischen, linearen oder verzweigten, Kohlenwasserstoffe weniger als fünfzehn Kohlenstoffatome oder mehr als neunzehn Kohlenstoffatome aufweisen.

3. Verfahren nach Anspruch 1, wobei die umgesetzte Mischung ($M_1$) für 100 % ihrer Masse umfasst:

- einen Massenanteil an verzweigten Alkanen, der größer als oder gleich 50 % und kleiner als oder gleich 100 % ist,
- einen Massenanteil an linearen Alkanen, der größer als oder gleich 0 % und kleiner als oder gleich 15 % ist,
- einen Massenanteil an Cycloalkanen, der größer als oder gleich 0 % und kleiner als oder gleich 35 % ist, und wobei zu 100 Massen-% der gesättigten cyclischen oder acyclischen, linearen oder verzweigten, Kohlenwasserstoffe von fünfzehn bis neunzehn Kohlenstoffatome aufweisen.

4. Verfahren nach Anspruch 2, wobei die umgesetzte Mischung ($M_1$) für 100 % ihrer Masse umfasst:

- einen Massenanteil an linearen Alkanen, der gleich 3,7 % ist,
- einen Massenanteil an Isoalkanen, der gleich 96 % ist, und
- einen Massenanteil an Cycloalkanen, der gleich 0,3 % ist.

5. Verfahren nach Anspruch 3, wobei die umgesetzte Mischung ($M_1$) für 100 % ihrer Masse umfasst:

- einen Massenanteil an linearen Alkanen, der gleich 13,20 % ist,
- einen Massenanteil an Isoalkanen, der gleich 55,00 % ist, und
- einen Massenanteil an Cycloalkanen, der gleich 31,80 % ist.

6. Emulsion für eine topische Verwendung des Öl-in-Wasser-Typs (E), **dadurch gekennzeichnet, dass** sie für 100 % ihrer Masse umfasst:

- von zu 50 Massen-% bis 90 Massen-% eine kosmetisch verträgliche wässrige Phase (A),
- von zu 10 Massen-% bis 50 Massen-% eine Fettphase (G), umfassend für 100 % ihrer Masse:

- von zu 10 Massen-% bis 50 Massen-%, insbesondere von zu 15 Massen-% bis 40 Massen-% eine Mischung ($M_1$) von gesättigten cyclischen oder acyclischen, linearen oder verzweigten Kohlenwasserstoffen die Mischung ($M_1$), umfassend für 100 % ihrer Masse entweder:

- einen Massenanteil an verzweigten Alkanen, der größer als oder gleich 80 % und kleiner als oder gleich 100 % ist,
- einen Massenanteil an linearen Alkanen, der größer als oder gleich 0 % und kleiner als oder gleich 15 % ist,

- einen Massenanteil an Cycloalkanen, der größer als oder gleich 0 % und kleiner als oder gleich 5 % ist,

und wobei von zu 95 Massen-% bis 100 Massen-% der gesättigten cyclischen oder acyclischen, linearen oder verzweigten, Kohlenwasserstoffe von fünfzehn bis neunzehn Kohlenstoffatome aufweisen, und bis zu 5 Massen-% der gesättigten cyclischen oder acyclischen, linearen oder verzweigten, Kohlenwasserstoffe weniger als fünfzehn Kohlenstoffatome oder mehr als neunzehn Kohlenstoffatome aufweisen;
oder:

- einen Massenanteil an verzweigten Alkanen, der größer als oder gleich 40 % und kleiner als oder gleich 100 % ist,
- einen Massenanteil an linearen Alkanen, der größer als oder gleich 0 % und kleiner als oder gleich 20 % ist,
- einen Massenanteil an Cycloalkanen, der größer als oder gleich 0 % und kleiner als oder gleich 40 % ist, und wobei zu 100 Massen-% der gesättigten cyclischen oder acyclischen, linearen oder verzweigten, Kohlenwasserstoffe von fünfzehn bis neunzehn Kohlenstoffatome aufweisen,
- von zu 0,5 Massen-% bis 15 Massen-% mindestens ein Tensid des Öl-in-Wasser-Typs,
- von zu 5 Massen-% bis 30 Massen-% mindestens ein Schutzmittel gegen ultraviolette Strahlung der Sonne,
- von zu 0 Massen-% bis 80 Massen-% mindestens ein Öl und/oder ein Wachs, wobei zu verstehen ist, dass mindestens ein solches Öl und/oder ein solches Wachs nicht der Definition der Mischung ($M_1$) entspricht;

**und dadurch, dass** sie frei von Sheabutter ist.

7. Emulsion für eine topische Verwendung des Öl-in-Wasser-Typs (E) nach Anspruch 6, **dadurch gekennzeichnet, dass** das Schutzmittel gegen ultraviolette Strahlung der Sonne ausgewählt ist aus den Elementen der Gruppe, bestehend aus Titandioxid, 2,4-Dihydroxybenzophenon, 2-(4-Diethylamino-2-hydroxybenzoyl)benzoesäurehexylester, 2,4-bis{[4-(2-Ethylhexyloxy)-2-hydroxy]phenyl}-6-(4-methoxyphenyl)-1,3,5-triazin, 2,4,6-tris[4-(2-ethylhexyloxycarbonyl)anilino]-1,3,5-Triazin und 2-Ethylhexyldimethoxybenzylidendioxoimidazolidinpropionat.

8. Öl-in-Wasser-Emulsion (E) nach einem der Ansprüche 6 oder 7, **dadurch gekennzeichnet, dass** die Mischung ($M_1$) für 100 % ihrer Masse umfasst:

- einen Massenanteil an verzweigten Alkanen, der größer als oder gleich 90 % und kleiner als oder gleich 100 % ist,
- einen Massenanteil an linearen Alkanen, der größer als oder gleich 0 % und kleiner als oder gleich 10 % ist,
- einen Massenanteil an Cycloalkanen, der größer als oder gleich 0 % und kleiner als oder gleich 1 % ist, wobei 95 Massen-% bis 100 Massen-% der gesättigten cyclischen oder acyclischen, linearen oder verzweigten, Kohlenwasserstoffe von fünfzehn bis neunzehn Kohlenstoffatome aufweisen, und bis zu 5 Massen-% der gesättigten cyclischen oder acyclischen, linearen oder verzweigten, Kohlenwasserstoffe weniger als fünfzehn Kohlenstoffatome oder mehr als neunzehn Kohlenstoffatome aufweisen.

9. Öl-in-Wasser-Emulsion (E) nach Anspruch 8,
**dadurch gekennzeichnet, dass** die Mischung ($M_1$) für 100 % ihrer Masse umfasst:

- einen Massenanteil an linearen Alkanen, der gleich 3,7 % ist;
- einen Massenanteil an Isoalkanen, der gleich 96 % ist;
- einen Massenanteil an Cycloalkanen, der gleich 0,3 % ist.

10. Emulsion für eine topische Verwendung des Öl-in-Wasser-Typs (E) nach einem der Ansprüche 6 oder 7, **dadurch gekennzeichnet, dass** die Mischung ($M_1$) für 100 % ihrer Masse umfasst:

- einen Massenanteil an verzweigten Alkanen, der größer als oder gleich 50 % ist,
- einen Massenanteil an linearen Alkanen, der größer als oder gleich 0 % und kleiner als oder gleich 15 % ist,
- einen Massenanteil an Cycloalkanen, der größer als oder gleich 0 % und kleiner als oder gleich 35 % ist,
wobei zu 100 Massen-% der gesättigten cyclischen oder acyclischen, linearen oder verzweigten, Kohlenwasser-

**EP 3 554 462 B1**

stoffe von fünfzehn bis neunzehn Kohlenstoffatome aufweisen.

11. Emulsion für eine topische Verwendung des Öl-in-Wasser-Typs (E) nach Anspruch 10, **dadurch gekennzeichnet, dass** die Mischung (M$_1$) für 100 % ihrer Masse umfasst:

- einen Massenanteil an linearen Alkanen, der gleich 13,20 % ist,
- einen Massenanteil an Isoalkanen, der gleich 55,00 % ist, und
- einen Massenanteil an Cycloalkanen, der größer als oder gleich 31,80 % ist.

12. Emulsion für eine topische Verwendung des Öl-in-Wasser-Typs (E) nach einem der Ansprüche 6 bis 11 zur Verwendung in einer therapeutischen Behandlungsmethode, die zum Vorbeugen und/oder Behandeln menschlicher Hautkrankheiten, die mit einer Aussetzung gegenüber ultravioletter Strahlung der Sonne verbunden sind, insbesondere Verbrennungen, Sonnenbrände, Erytheme und Hautkrebs, bestimmt ist.

**Claims**

1. Method for improving the sensory and/or aesthetic properties of an oil-in-water type emulsion (E$_0$), said sensory properties being the spreading properties, the consistency properties and the richness properties of said emulsion for topical use (E$_0$), **characterized in that** an effective quantity of a mixture (M$_1$) of cyclic or acyclic, linear or branched saturated hydrocarbons is incorporated therein, comprising for 100% of its mass either:

- A mass proportion of branched alkanes greater than or equal to 80% and less than or equal to 100%;
- A mass proportion of linear alkanes greater than or equal to 0% and less than or equal to 15%;
- A mass proportion of cycloalkanes greater than or equal to 0% and less than or equal to 5%; and from 95% by mass to 100% by mass of said cyclic or acyclic, linear or branched saturated hydrocarbons comprising from fifteen to nineteen carbon atoms, and up to 5% by mass of said cyclic or acyclic, linear or branched saturated hydrocarbons comprising fewer than fifteen carbon atoms or more than nineteen carbon atoms or:

- A mass proportion of branched alkanes greater than or equal to 40% and less than or equal to 100%,
- A mass proportion of linear alkanes greater than or equal to 0% and less than or equal to 20%,
- A mass proportion of cycloalkanes greater than or equal to 0% and less than or equal to 40% and 100% by mass of said cyclic or acyclic, linear or branched saturated hydrocarbons comprising from fifteen to nineteen carbon atoms;
**and in that** said emulsion (E$_0$) is free from shea butter.

2. Method as defined in claim 1, wherein the mixture (M$_1$) used comprises for 100% of its mass:

- A mass proportion of branched alkanes greater than or equal to 90% and less than or equal to 100%,
- A mass proportion of linear alkanes greater than or equal to 0% and less than or equal to 10%,
- A mass proportion of cycloalkanes greater than or equal to 0% and less than or equal to 1%;
and wherein from 95% by mass to 100% by mass of said cyclic or acyclic, linear or branched saturated hydrocarbons comprise from fifteen to nineteen carbon atoms, and up to 5% by mass of said cyclic or acyclic, linear or branched saturated hydrocarbons comprise fewer than fifteen carbon atoms or more than nineteen carbon atoms.

3. Method as defined in claim 1, wherein the mixture (M$_1$) used comprises for 100% of its mass:

- A mass proportion of branched alkanes greater than or equal to 50% and less than or equal to 100%,
- A mass proportion of linear alkanes greater than or equal to 0% and less than or equal to 15%,
- A mass proportion of cycloalkanes greater than or equal to 0% and less than or equal to 35%,
and wherein 100% by mass of said cyclic or acyclic, linear or branched saturated hydrocarbons comprise from fifteen to nineteen carbon atoms.

4. Method as defined in claim 2, wherein the mixture (M$_1$) used comprises for 100% of its mass:

- A mass proportion of linear alkanes equal to 3.7%,
- A mass proportion of isoalkanes equal to 96%, and

23

- A mass proportion of cycloalkanes equal to 0.3%.

5. Method as defined in claim 3, wherein the mixture ($M_1$) used comprises for 100% of its mass:

- A mass proportion of linear alkanes equal to 13.20%,
- A mass proportion of isoalkanes equal to 55.00% and
- A mass proportion of cycloalkanes equal to 31.80%.

6. Oil-in-water type emulsion for topical use (E), **characterized in that** it comprises for 100% of its mass:

- From 50% to 90% by mass of a cosmetically acceptable aqueous phase (A),
- From 10% to 50% by mass of a fatty phase (G) comprising for 100% of its mass:

    - From 10% to 50% by mass, more particularly from 15% to 40% by mass of a mixture ($M_1$) of cyclic or acyclic, linear or branched saturated hydrocarbons, said mixture ($M_1$) comprising for 100% of its mass either:

        - A mass proportion of branched alkanes greater than or equal to 80% and less than or equal to 100%,
        - A mass proportion of linear alkanes greater than or equal to 0% and less than or equal to 15%,
        - A mass proportion of cycloalkanes greater than or equal to 0% and less than or equal to 5%,

        and from 95% by mass to 100% by mass of said cyclic or acyclic, linear or branched saturated hydrocarbons comprising from fifteen to nineteen carbon atoms, and up to 5% by mass of said cyclic or acyclic, linear or branched saturated hydrocarbons comprising fewer than fifteen carbon atoms or more than nineteen carbon atoms;
        or:

            - A mass proportion of branched alkanes greater than or equal to 40% and less than or equal to 100%,
            - A mass proportion of linear alkanes greater than or equal to 0% and less than or equal to 20%,
            - A mass proportion of cycloalkanes greater than or equal to 0% and less than or equal to 40% and 100% by mass of said cyclic or acyclic, linear or branched saturated hydrocarbons comprising from fifteen to nineteen carbon atoms,
        - From 0.5% to 15% by mass of at least one oil-in-water type surfactant,
        - From 5% to 30% by mass of at least one agent for protection against ultraviolet radiation from the sun,
        - From 0% to 80% by mass of at least one oil and/or wax, it being understood that at least one such oil and/or wax does not meet the definition of mixture ($M_1$);

    **and in that** it is free of shea butter.

7. Oil-in-water emulsion for topical use (E) as defined in claim 6, **characterized in that** the agent for protection against ultraviolet radiation from the sun is selected from the elements of the group consisting of titanium dioxide, 2,4-dihydroxybenzophenone, 2-(4-diethylamino-2-hydroxybenzoyl) benzoic acid hexyl ester, 2,4-bis{[4-(2-ethylhexyloxy)-2-hydroxy] phenyl}-6-(4-methoxyphenyl)-1,3,5-triazine, 2,4,6-tris[4-(2-ethylhexyloxy carbonyl) anilino]-1,3,5-triazine and 2-ethylhexyl dimethoxy benzylidene dioxoimidazolidine propionate.

8. Oil-in-water emulsion (E) as defined in either claim 6 or claim 7, **characterized in that** said mixture ($M_1$) comprises for 100% of its mass:

- A mass proportion of branched alkanes greater than or equal to 90% and less than or equal to 100%,
- A mass proportion of linear alkanes greater than or equal to 0% and less than or equal to 10%,
- A mass proportion of cycloalkanes greater than or equal to 0% and less than or equal to 1%, from 95% by mass to 100% by mass of said cyclic or acyclic, linear or branched saturated hydrocarbons comprising from fifteen to nineteen carbon atoms and up to 5% by mass of said cyclic or acyclic, linear or branched saturated hydrocarbons comprising fewer than fifteen carbon atoms or more than nineteen carbon atoms.

9. Oil-in-water emulsion (E) as defined in claim 8, **characterized in that** said mixture ($M_1$) comprises for 100% of its mass:

- A mass proportion of linear alkanes equal to 3.7%;
- A mass proportion of isoalkanes equal to 96%;
- A mass proportion of cycloalkanes equal to 0.3%.

10. Oil-in-water type emulsion for topical use (E) as defined in either claim 6 or claim 7, **characterized in that** said mixture ($M_1$) comprises for 100% of its mass:

- A mass proportion of branched alkanes greater than or equal to 50%,
- A mass proportion of linear alkanes greater than or equal to 0% and less than or equal to 15%,
- A mass proportion of cycloalkanes greater than or equal to 0% and less than or equal to 35%,
100% by mass of said cyclic or acyclic, linear or branched saturated hydrocarbons comprising from fifteen to nineteen carbon atoms.

11. Oil-in-water type emulsion for topical use (E) as defined in claim 10, **characterized in that** said mixture ($M_1$) comprises for 100% of its mass:

- A mass proportion of linear alkanes equal to 13.20%,
- A mass proportion of isoalkanes equal to 55.00% and
- A mass proportion of cycloalkanes greater than or equal to 31.80%.

12. Oil-in-water type emulsion for topical use (E) as defined in any of claims 6 to 11, for use in a therapeutic treatment method for preventing and/or treating human skin diseases related to exposure to ultraviolet radiation from the sun, more particularly burns, sunburn, erythema, skin cancers.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2644188 A1
- WO 2011065771 A
- WO 2008155060 A
- EP 0971683 A

**Littérature non-brevet citée dans la description**

- **AGRANA**. *Winter Comfort Eco-conscious Hand Cream MAISITA 9040*
- **JENNIFER NOVOSELETSKY**. *Seppic introduces Emollient Ranges by the Double*, 17 April 2016, www.cosmeticsandtoiletries.com
- **MICHEL** ; **IRENE ASH**. Thesaurus of Chemical Products. Chemical Publishing Co, Inc., 1986, vol. I, 211